# EUROPEAN PATENT APPLICATION

(11) **EP 3 858 401 A1**
(43) Date of publication of application: **04.08.2021**
(21) Application number: 19881520.1
(22) Date of filing: 26.09.2019
(51) Int. Cl.: A61M 5/142

(54) **MEDICAL DEVICE**

(30) Priority: 07.11.2018 JP 2018209904
(71) Applicant: Terumo Kabushiki Kaisha, Tokyo 151-0072 (JP)
(72) Inventor: MIZUTANI, Motoki, Nakakoma-gun, Yamanashi 409-3853 (JP); UCHIYAMA, Joji, Ashigarakami-gun, Kanagawa 259-0151 (JP)
(74) Representative: Casalonga
(86) International application number: PCT/JP2019/038040
(87) International publication number: WO 2020/095565

(57) **Abstract**

This disclosure can provide a medical device capable of enhancing a fixing force between a housing and an adhesion portion and an adhesive force between the adhesion portion and a living body surface. A drug solution delivery apparatus (1) includes: a housing (10) that can be attached to a living body; a sheet-shaped adhesion portion (16) provided on a bottom surface (11b) which is disposed on a living body side in the housing; and a fixing region (110) to which the housing and the adhesion portion are fixed. The housing includes a fitting hole (12) which is formed in the bottom surface of the housing to penetrate the bottom surface. The adhesion portion includes an adhesive portion (16c) formed in at least a part of a bottom surface (16b) disposed to face a living body. The fixing region includes a welded portion (111) formed near the hole portion and a non-welded portion (112) formed at a position adjacent to the welded portion.

## Description

### Technical Field

This disclosure relates to a medical device.

### Background Art

In the related art, as an apparatus for delivering a drug solution such as insulin, there has been known a portable drug solution delivery apparatus for delivering a drug solution continuously or intermittently in a state of being attached to skin of a patient or a subject to be delivered (for example, see PTL 1).

The drug solution delivery apparatus described above includes a housing and an adhesion portion (adhesive portion) on a surface (for example, a bottom surface) of the housing which faces a living body surface. The adhesion portion maintains the state in which the apparatus is attached to a living body.

### Citation List

### Patent Literature

PTL 1: JP-T-2010-525868

### Summary of Invention

### Technical Problem

When the adhesion portion is formed on the housing, for example, an adhesive tape having adhesive surfaces on both surfaces of a surface on a housing side and a surface on a living body surface side can be used. However, when the adhesive tape is used, a fixing force between the housing and the adhesive tape may not be maintained only by an adhesive force of the adhesive tape. In order to solve such a problem, for example, a method for welding and fixing an adhesive tape to a housing is considered.

However, when the adhesive tape is welded and fixed to the housing, the adhesive force to the living body surface may be reduced due to an influence of heat generated at the time of welding in a portion where the adhesive tape is welded and fixed. In addition, a hole or the like penetrating the housing is formed in the housing and it is difficult to secure a large contact area between the housing and the adhesive tape around the hole. Thus, the adhesive tape is likely to be detached from the housing.

An object of this disclosure is to provide a medical device capable of enhancing a fixing force between a housing and an adhesion portion and an adhesive force between the adhesion portion and a living body surface.

### Solution to Problem

In order to achieve the above object, a medical device according to this disclosure includes: a housing attachable to a living body; a sheet-shaped adhesion portion provided on one surface on a living body side of the housing; and a fixing region to which the housing and the adhesion portion are fixed. The housing includes a hole portion formed in the one surface of the housing and penetrating the one surface. The adhesion portion includes an adhesive portion formed in at least a part of a surface facing the living body. The fixing region includes a welded portion formed near the hole portion and a non-welded portion formed at a position adjacent to the welded portion.

### Advantageous Effect of Invention

According to this disclosure, a medical device that enhances a fixing force between a housing and an adhesion portion and an adhesive force between the adhesion portion and a living body surface can be provided.

### Brief Description of Drawings

[Fig. 1] Fig. 1 is an exploded perspective view showing a medical device (drug solution delivery apparatus) according to an embodiment.
[Fig. 2] Fig. 2 is a perspective view of the medical device.
[Fig. 3] Fig. 3 is a perspective view of the medical device when viewed from a bottom surface side.
[Fig. 4] Fig. 4 is a perspective view showing a housing and a puncture apparatus.
[Fig. 5] Fig. 5 is a perspective view showing the housing and the puncture apparatus.
[Fig. 6] Fig. 6 is a plan view of the housing.
[Fig. 7] Fig. 7 is a plan view of a housing according to a first modification.
[Fig. 8] Fig. 8 is a plan view of a housing according to a second modification.
[Fig. 9] Fig. 9 is a plan view of a housing according to a third modification.
[Fig. 10] Fig. 10 is a plan view of a housing according to a fourth modification.
[Fig. 11] Fig. 11 is a cross-sectional view taken along arrows 11-11 shown in Fig. 10.
[Fig. 12] Fig. 12 is a plan view of a housing according to a fifth modification.
[Fig. 13] Figs. 13(A), 13(B), and 13(C) show examples of shapes of other welded portions.
[Fig. 14] Figs. 14(A), 14(B), 14(C), and 14(D) show examples of shapes of other welded portions.

### Description of Embodiments

Hereinafter, embodiments according to this disclosure will be described with reference to the drawings. The following description does not limit the technical scope and the meaning of terms described in the claims. Further, dimensional ratios in the drawings are exaggerated for convenience of description and may differ from the actual ratios.

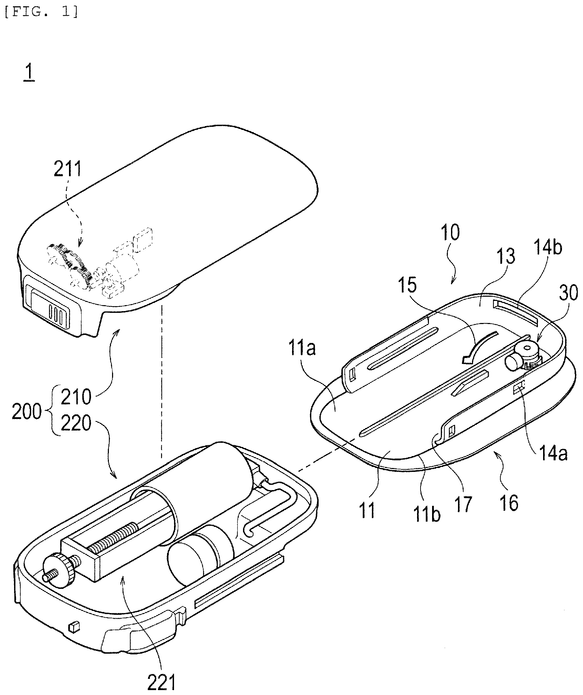

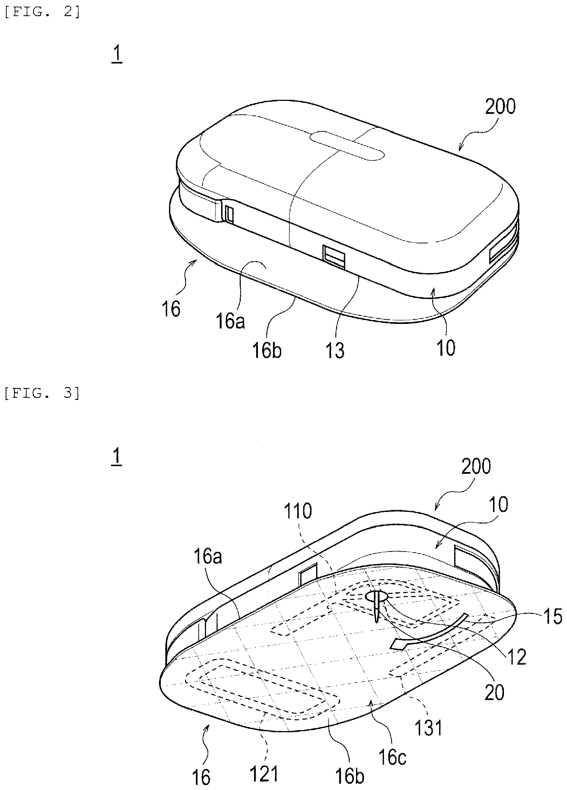

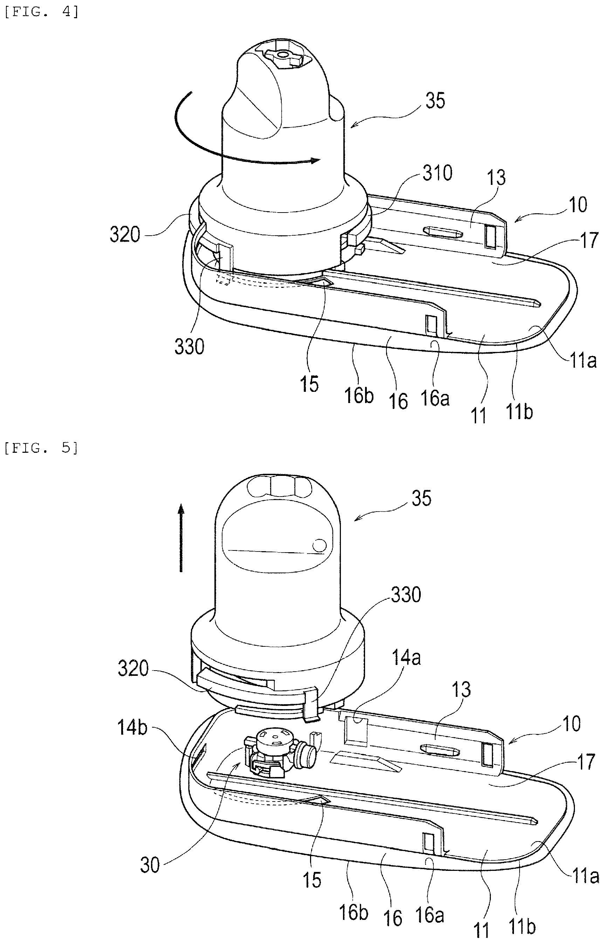

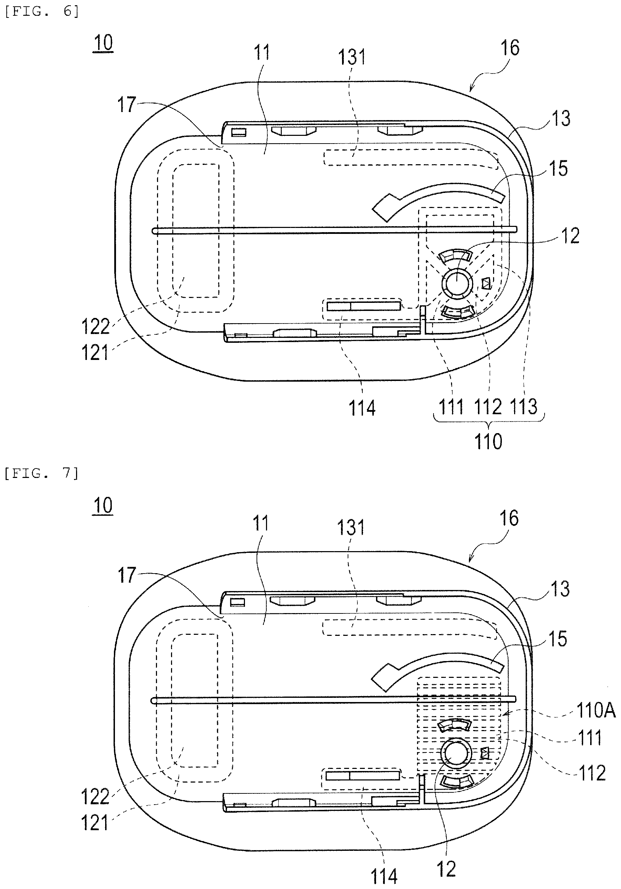

Fig. 1 is an exploded perspective view of a drug solution delivery apparatus (medical device) 1 according to the present embodiment, Figs. 2 and 3 are perspective views of the drug solution delivery apparatus (medical device) 1, Figs. 4 and 5 show a housing 10 and a puncture apparatus 35, and Fig. 6 is a plan view of the housing 10.

As shown in Fig. 1, the drug solution delivery apparatus 1 serves as a portable insulin delivery apparatus that delivers insulin serving as a drug solution into a living body of a diabetic patient who is a user. The drug solution is not limited to the insulin, and may be other drug solutions.

As shown in Figs. 1, 3, and 6, the drug solution delivery apparatus 1 generally includes the housing 10 attachable to a living body, a sheet-shaped adhesion portion 16 provided on a bottom surface (corresponding to one surface) 11b on a living body side of the housing 10, and a fixing region 110 to which the housing 10 and the adhesion portion 16 are fixed.

The housing 10 has a fitting hole 12 (corresponding to a hole portion) formed in the bottom surface 11b of the housing 10 and penetrating the bottom surface. The adhesion portion 16 includes an adhesive portion 16c formed on at least a part of the bottom surface 16b (corresponding to one surface) facing a living body. As shown in Figs. 3 and 6, the fixing region 110 includes welded portions 111 formed near the fitting hole 12 and non-welded portions 112 formed at positions adjacent to the welded portions 111.

### <Drug solution delivery apparatus>

As shown in Fig. 1, the drug solution delivery apparatus 1 includes a liquid delivering mechanism 200 connectable to and separable from the housing 10.

The liquid delivering mechanism 200 is not particularly limited as long as the liquid delivering mechanism can deliver a drug solution into a living body. For example, a liquid delivering mechanism including a reuse portion 210 including a drive mechanism 211 or the like that generates a drive force for driving a member necessary for a liquid delivering operation, and a disposable portion 220 including a drug solution storage unit 221 or the like filled with insulin and connectable to and separatable from the reuse portion 210 can be used. As shown in Figs. 2 and 3, the user can connect the liquid delivering mechanism 200 to the housing 10 in a state where the reuse portion 210 and the disposable unit 220 are connected to each other.

### <Puncture apparatus>

Figs. 4 and 5 show the puncture apparatus 35. The puncture apparatus 35 is configured to connect a delivery port portion 30 that delivers the drug solution into the living body to the housing 10, and to puncture a puncture needle 20 (see Fig. 3) that punctures the living body.

As shown in Figs. 4 and 5, the user connects the delivery port portion 30 to the housing 10 by using the puncture apparatus 35 before connecting the liquid delivering mechanism 200 to the housing 10. The delivery port portion 30 is fixed by being fitted into the fitting hole 12 (see Fig. 6) of the housing 10. The user uses the puncture apparatus 35 to protrude the puncture needle 20 from a bottom surface 11b side of the housing 10 and puncture the living body with the puncture needle 20. The puncture needle 20 can be constituted by, for example, a double needle including a cannula having an inner cavity and an inner needle inserted through the cannula.

As shown in Figs. 4 and 5, the puncture apparatus 35 connects the delivery port portion 30 to the housing 10 and protrudes the puncture needle 20 by a rotation operation relative to the housing 10.

As shown in Figs. 4 and 5, the puncture apparatus 35 includes a first guide portion 310, a second guide portion 320, and a third guide portion 330 that guide the operation of the puncture apparatus 35 when the puncture apparatus 35 is rotated. A side wall 13 of the housing 10 is formed with a first hole portion 14a through which the first guide portion 310 is inserted and a second hole portion 14b through which the second guide portion 320 is inserted. A third hole portion 15 into which the third guide portion 330 is inserted is formed in a bottom portion 11 of the housing 10.

When the puncture apparatus 35 is rotated, the guide portions 310, 320, 330 of the puncture apparatus 35 are inserted into and engaged with the hole portions 14a, 14b, 15, respectively. As shown in Fig. 4, the user can guide the rotation of the puncture apparatus 35 by the guide portions 310, 320, 330 while rotating the puncture apparatus 35. Therefore, the user can rotate the puncture apparatus 35 in parallel with the bottom portion 11 of the housing 10. As shown in Fig. 5, after the puncture apparatus 35 is rotated by a certain amount, the engagement between the hole portions 14a, 14b, 15 and the guide portions 310, 320, 330 is released. The user can separate the puncture apparatus 35 from the housing 10 by lifting the puncture apparatus 35 after the engagement between the hole portions 14a, 14b, 15 and the guide portions 310, 320, 330 is released.

The puncture apparatus 35 can be connected to the housing 10 before the liquid delivering mechanism 200 is connected to the housing 10. The user attaches the housing 10 to a living body surface of the user by using the adhesion portion 16 formed on the housing 10. The user connects the delivery port portion 30 to the housing 10 and punctures the living body with the puncture needle 20 by using the puncture apparatus 35 in a state where the housing 10 is attached to the living body surface. After separating the puncture apparatus 35 from the housing 10, the user connects the liquid delivering mechanism 200 to the housing 10 as shown in Figs. 2 and 3. When connecting the liquid delivering mechanism 200 to the housing 10, the user fluidly communicates the delivery port portion 30, the cannula of the puncture needle 20, and the drug solution storage unit 221. The user can deliver the drug solution into the living body by operating a predetermined controller to drive the drive mechanism 211.

### <Housing>

As shown in Figs. 1 and 6, the housing 10 includes the bottom portion 11 to be attached to the living body via the adhesion portion 16, the side wall 13 surrounding a part of a periphery of the bottom portion 11, and insertion portions 17 for insertion of the liquid delivering mechanism 200 into the housing 10 when connecting the liquid delivering mechanism 200 to the housing 10.

As shown in Fig. 6, the bottom portion 11 includes a placement surface (upper surface) 11a on which the liquid delivering mechanism 200 can be placed, the bottom surface 11b located on a back surface side of the placement surface 11a, the fitting hole (hole portion) 12 into which the delivery port portion 30 can be fitted, and the third hole portion 15 into which the third guide portion 330 of the puncture apparatus 35 is inserted.

The housing 10 has a substantially elliptical planar shape. The fitting hole 12 has a substantially circular planar shape. The insertion portions 17 are constituted by opening portions formed by cutting out a part of the side wall 13. The third hole portion 15 has a planar shape extending in a substantially arc shape along a longitudinal direction (left-right direction in Fig. 6) of the housing 10. The housing 10 can be formed of a resin material such as polypropylene, polyethylene terephthalate, polymethyl methacrylate, or polycarbonate.

The fitting hole 12 penetrates the bottom portion 11 of the housing 10 in a thickness direction. Similarly, the third hole portion 15 penetrates the bottom portion 11 of the housing 10 in the thickness direction.

As shown in Fig. 6, the insertion portions 17 are formed on one end portion side in the longitudinal direction of the housing 10 in which the fitting hole 12 is disposed and the other portion side opposite to the one end portion side.

As shown in Figs. 2, 3, and 6, the adhesion portion 16 includes an upper surface 16a disposed on the bottom surface 11b side of the housing 10, a bottom surface 16b located on a back surface side of the upper surface 16a, and the adhesive portion 16c (simply shown by oblique lines in Fig. 3) formed on the bottom surface 16b.

The adhesion portion 16 is larger than the housing 10 to protrude from an outer periphery of the housing 10 in the plan view shown in Fig. 6. The upper surface 16a of the adhesion portion 16 is fixed to the bottom surface 11b of the housing 10 by welding. With such a structure, an adhesive area is increased, peeling generated from the outer periphery of the housing 10 can be prevented, and the state where the housing 10 is attached to the living body surface can be stably maintained.

A hole portion through which the puncture needle 20 is inserted is formed in the adhesion portion 16 at a position corresponding to the fitting hole 12 of the housing 10. A hole portion through which the third guide portion 330 of the puncture apparatus 35 is inserted is formed in the adhesion portion 16 at a position corresponding to the third hole portion 15 of the housing 10.

The adhesion portion 16 can be constituted by an adhesive member (single-sided adhesive tape) including a sheet-shaped base material and the adhesive portion 16c provided on a bottom surface of the base material (the bottom surface 16b of the adhesion portion 16). The base material can be constituted by, for example, a polyethylene film, a polyester nonwoven fabric, a rayon nonwoven fabric, or a resin coated cotton fabric. The adhesive portion 16c can be constituted by an adhesive, for example, an acrylic adhesive, a silicone adhesive, or a gel adhesive. The adhesion portion 16 may be provided with a release paper or the like that covers the bottom surface 16b of the adhesion portion 16 until the housing 10 is attached to the living body. The adhesion portion 16 is not limited to a single-sided tape and may be constituted by a double-sided tape, an adhesive to be applied to the base material, or the like, but the single-sided adhesive tape is preferable due to the structure protruding from the outer periphery of the housing 10.

In the fixing region 110, as shown in Fig. 6, the welded portions 111 and the non-welded portions 112 surround a periphery of the fitting hole 12. The welded portions 111 and the non-welded portions 112 are alternately disposed along the outer periphery of the fitting hole 12. In the present specification, the welded portions 111 refer to portions (portions indicated by broken lines in Fig. 6) where the housing 10 and the adhesion portion 16 are welded to each other in the fixing region 110, and the non-welded portions 112 refer to portions (regions) where the housing 10 and the adhesion portion 16 are not welded to each other in the fixing region 110.

As shown in Fig. 6, other welded portions 113, 114 may be formed close to the welded portions 111 and the non-welded portions 112 in the fixing region 110. The welded portion 113 extends to connect the linear welded portions 111 around the fitting hole 12. The welded portion 113 extends from the fitting hole 12 to a vicinity of the third hole portion 15. The welded portion (corresponding to an auxiliary welded portion) 114 extends substantially linearly from the welded portion 111 along the longitudinal direction of the housing 10. Each of the welded portions 113, 114 may be or may not continuous with the welded portions 111.

In the fixing region 110, the welded portions 111 are formed in four linear patterns extending radially from the fitting hole 12. The non-welded portions 112 formed between the welded portions 111 have a substantially triangular planar shape surrounded by the welded portions 111 and the welded portion 113. The pattern of the welded portions 111, 113, 114 and a shape of the non-welded portions 112 are not particularly limited.

When the adhesion portion 16 and the housing 10 are fixed by welding, a part of the housing 10 where the fitting hole 12 is formed cannot be welded. Therefore, in the vicinity of the fitting hole 12, a fixing force between the adhesion portion 16 and the housing 10 is reduced. As a measure against such a problem, it is considered that the fixing force between the adhesion portion 16 and the housing 10 can be improved by forming a welded portion to surround the entire periphery of the fitting hole 12. However, when the adhesive portion 16c of the adhesion portion 16 is affected by heat applied when the welded portion is formed, an adhesive force of the adhesive portion 16c is reduced. Therefore, when the housing 10 is attached to the living body surface of the user, the state where the housing 10 is attached to the living body surface via the adhesion portion 16 may not be stably maintained. When the fixing force between the adhesion portion 16 and the housing 10 and/or the adhesive force of the adhesive portion 16c decreases in the vicinity of the fitting hole 12, the puncture needle 20 punctured into the living body may come off, and the fluid communication at the delivery port portion 30 serving as a connection portion may be released.

In the drug solution delivery apparatus 1 according to the present embodiment, since both the welded portions 111 and the non-welded portions 112 are formed around the fitting hole 12 in the fixing region 110, it is possible to prevent a significant decrease in the adhesive force of the adhesive portion 16c of the adhesion portion 16 and increase the fixing force for fixing the housing 10 to the adhesion portion 16.

As shown in Fig. 6, the drug solution delivery apparatus 1 further includes a welded portion (corresponding to another welded portion) 121 in which the housing 10 and the adhesion portion 16 are welded to each other in a vicinity of the insertion portions 17. When the liquid delivering mechanism 200 is connected to the housing 10, the user inserts the liquid delivering mechanism 200 into the placement surface 11a of the housing 10 from an insertion portions 17 side. Therefore, an external force is more likely to be applied to the vicinity of the insertion portions 17 than to other portions of the housing 10. In the drug solution delivery apparatus 1, by providing the welded portion 121, it is possible to suitably prevent the fixing between the housing 10 and the adhesion portion 16 from being released in the vicinity of the insertion portions 17. In addition, the welded portion 121 can prevent the housing 10 and the adhesion portion 16 from being separated from each other even when the liquid delivering mechanism 200 is detached from the housing 10.

The welded portion 121 is formed in a frame shape in a direction intersecting the longitudinal direction of the housing 10. A non-welded portion 122 is formed in a region surrounded by the welded portion 121. The specific shapes of the welded portion 121 and the non-welded portion 122 are not particularly limited.

As shown in Fig. 6, the drug solution delivery apparatus 1 further includes a welded portion 131 formed on a side wall 13 side (an outer peripheral side of the housing 10) relative to the third hole portion 15. The welded portion 131 extends along the longitudinal direction of the housing 10.

The fixing region 110 is preferably formed, for example, in a range of 0% to 50% of an entire length of the housing 10 along the longitudinal direction of the housing 10 from one end portion (an end portion on a right side in Fig. 6) of the housing 10. The welded portion 121 is preferably formed, for example, in a range of 10% to 40%, and more preferably in a range of 15% to 35%, of the entire length of the housing 10 along the longitudinal direction of the housing 10 from the other end portion (an end portion on a left side in Fig. 6) of the housing 10. In the present embodiment, in a region (non-welded region) between the fixing region 110 and the welded portion 121, the adhesive force of the adhesive portion 16c of the adhesion portion 16 is larger than that of other portions. Therefore, by setting the range in which the fixing region 110 is formed and the position at which the welded portion 121 is formed as described above, it is possible to prevent the adhesive force of the adhesive portion 16c from becoming excessively small. When the welded portion 131 is formed in the drug solution delivery apparatus 1, the welded portion 131 can be formed in the same range as the fixing region 110.

In addition, each of the welded portions 111, 113, 114, 121, 131 can be formed by continuously connecting any of the welded portions. Other welded portions (not shown) (for example, welded portions extending linearly in the longitudinal direction of the housing 10 or in the direction intersecting the longitudinal direction) may be formed, and the welded portions 111, 113, 114, 121, 131 may be selectively connected to each other via corresponding welded portions.

A method for forming each of the welded portions 111, 113, 114, 121, 131 is not particularly limited, and for example, a known method such as thermal welding by embossing, ultrasound welding, laser welding, vibration welding (horn welding), hot air, or spin welding can be performed. In particular, when each of the welded portions 111, 113, 114, 121, 131 is formed by the thermal welding, since a mold for applying heat is used, it is possible to perform welding with high reproducibility in alignment. In addition, by changing the mold, it is possible to easily change a welding pattern. Thus, welding having various patterns can be performed in accordance with the housing 10.

As described above, the drug solution delivery apparatus 1 according to the present embodiment includes the housing 10 attachable to a living body, the sheet-shaped adhesion portion 16 provided on the bottom surface 11b of the housing 10, and the fixing region 110 to which the housing 10 and the adhesion portion 16 are fixed. The housing 10 includes the fitting hole 12 formed in the bottom surface 11b of the housing 10 and penetrating the bottom surface 11b. The adhesion portion 16 includes the adhesive portion 16c formed on at least a part of the bottom surface 16b of the adhesion portion 16. The fixing region 110 includes the welded portions 111 formed near the fitting hole 12 and the non-welded portions 112 formed at positions adjacent to the welded portions 111.

According to the drug solution delivery apparatus 1 described above, the fixing force between the adhesion portion 16 and the housing 10 can be increased by the welded portions 111 provided in the fixing region 110. In addition, the non-welded portions 112 provided in the fixing region 110 can prevent the adhesive force of the adhesive portion 16c from being excessively reduced in the fixing region 110. Therefore, in the drug solution delivery apparatus 1, the fixing force between the housing 10 and the adhesion portion 16 and the adhesive force between the adhesion portion 16 and the living body surface are increased.

The fixing region 110 surrounds the periphery of the fitting hole 12, and the welded portions 111 and the non-welded portions 112 are alternately disposed along the outer periphery of the fitting hole 12. Since both the welded portions 111 and the non-welded portions 112 are formed around the fitting hole 12 in the fixing region 110, it is possible to prevent a significant decrease in the adhesive force of the adhesive portion 16c of the adhesion portion 16 and increase the fixing force for fixing the housing 10 to the adhesion portion 16. As a result, the fixing force between the housing 10 and the adhesion portion 16 around the fitting hole 12 and the adhesive force between the adhesion portion 16 and the living body surface are maintained, so that a state of the puncture needle 20 puncturing the living body is more reliable.

The housing 10 further includes the welded portion 113 formed at a position different from the welded portions 111 along one direction of the housing 10. Therefore, the fixing force between the housing 10 and the adhesion portion 16 can be further increased.

The housing 10 includes the insertion portions 17 for insertion of the liquid delivering mechanism 200 to be connected to the housing 10 into the housing 10. The housing 10 includes the welded portion 121 in which the housing 10 and the adhesion portion 16 are welded to each other in the vicinity of the insertion portions 17. Therefore, when the liquid delivering mechanism 200 is attached to or detached from the housing 10, it is possible to prevent the adhesion portion 16 from being peeled off from the housing 10 or the adhesion portion 16 from being twisted.

The medical device according to this disclosure has been described above through the embodiment, but the invention is not limited to the contents described in the specification, and can be appropriately changed based on the description of the claims.

For example, the patterns (shapes) of the welded portions 111 and the non-welded portions 112 provided in the fixing region 110 are not limited to the patterns shown in Fig. 6.

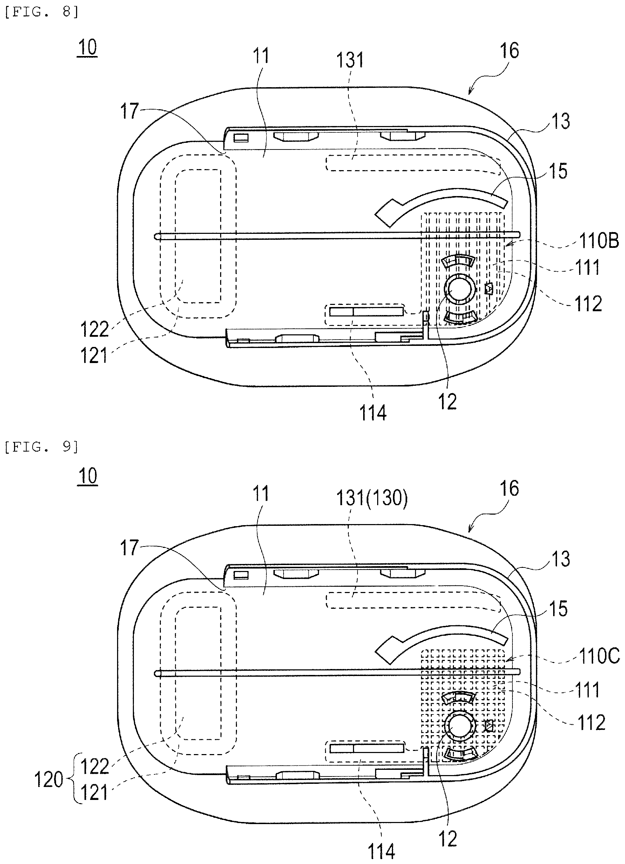

As shown in Fig. 7, the welded portions 111 and the non-welded portions 112 may be formed in parallel linear patterns in which the welded portions 111 and the non-welded portions 112 are spaced apart from each other in a fixing region 110A. The welded portions 111 and the non-welded portions 112 can be formed, for example, in a shape linearly extending in the longitudinal direction (left-right direction in Fig. 7) of the housing 10. Further, as shown in Fig. 8, the welded portions 111 and the non-welded portions 112 may be formed in a shape linearly extending in a direction (vertical direction in Fig. 8) intersecting the longitudinal direction of the housing 10 in a fixing region 110B. As shown in Fig. 9, the welded portions 111 and the non-welded portions 112 may be formed in a lattice-shaped pattern in which the welded portions 111 and the non-welded portions 112 intersect with each other in a fixing region 110C.

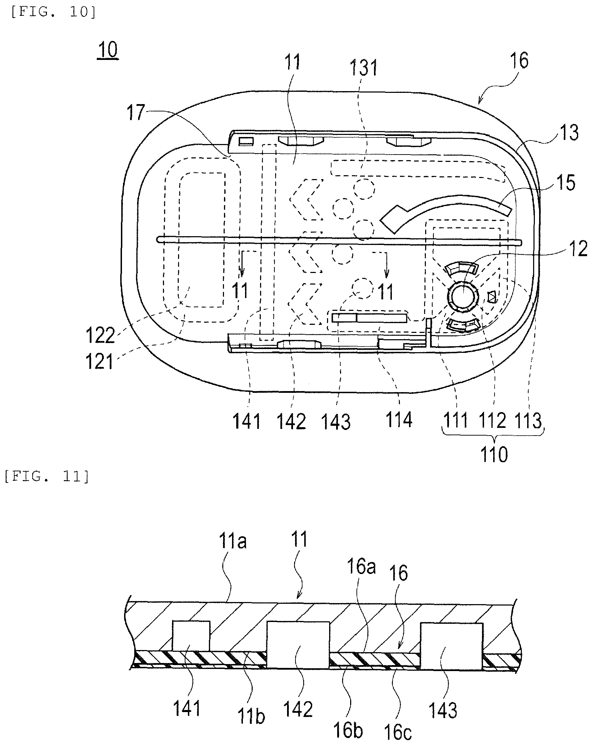

As shown in Figs. 10 and 11, in the region of the housing 10 where the welded portions 111, 113, 114, 121, 131 are not formed, for example, a passage 141 that allows air to flow on the bottom surface 11b side of the housing 10, groove portions 142, 143 that reduce a contact area between the living body surface and the bottom surface 11b of the housing 10, and the like may be formed. The passage 141 prevents a user from feeling uncomfortable due to an increase in humidity between the housing 10 and the living body surface by flowing air on the bottom surface 11b side of the housing 10. The groove portions 142, 143 can reduce an influence of the adhesive portion 16c on skin of the user by reducing the contact area between the living body surface and the bottom surface 11b of the housing 10.

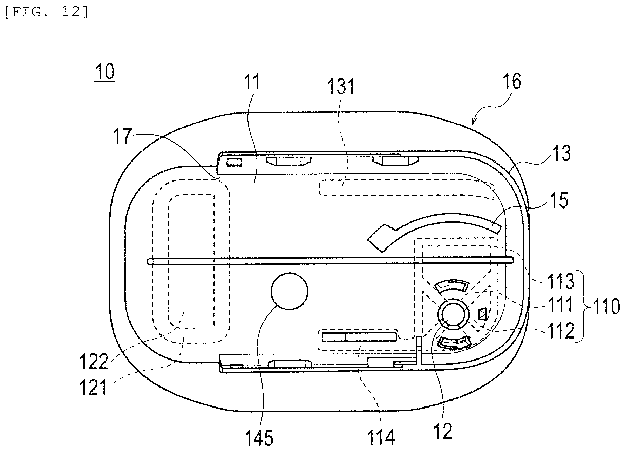

As shown in Fig. 12, for example, a vent hole 145 penetrating the bottom portion 11 of the housing 10 may be formed in the region of the housing 10 where the welded portions 111, 113, 114, 121, 131 are not formed. By forming the vent hole 145, water vapor or the like can be released from the space between the housing 10 and the living body surface, so that the influence on the skin of the user can be reduced.

In addition, by forming the passage 141, the groove portions 142, 143, the vent hole 145, and the like in the region where the welded portions 111, 113, 114, 121, 131 are not formed in the housing 10, magnitude of the adhesive force of the adhesive portion 16c in the region can be adjusted.

Each of the groove portions 142, 143 can be formed by blasting, surface processing using a mold, or the like. In addition, the passage 141 can be formed by the surface processing using a mold or the like. Specific shapes, positions, and the like of the passage 141, the groove portions 142, 143, and the vent hole 145 are not limited to those described with reference to the drawings.

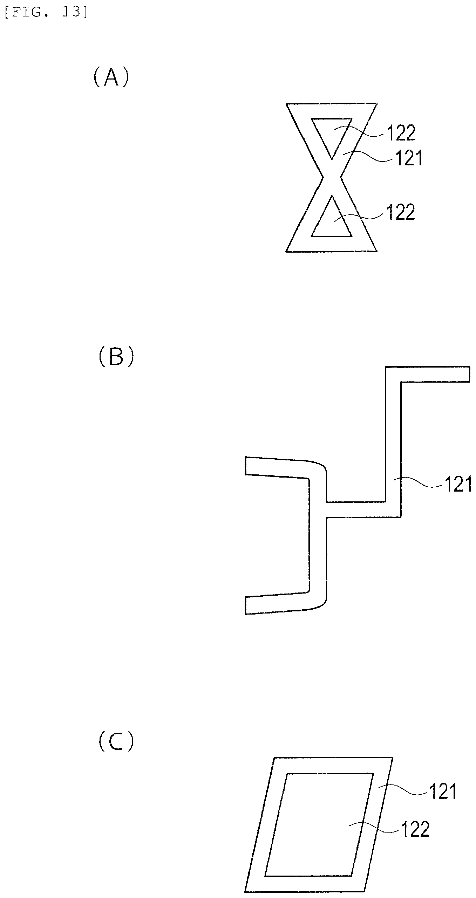

The pattern (shape) of the welded portion 121 formed in the vicinity of the insertion portions 17 is not limited to the pattern shown in Fig. 6. For example, as shown in Fig. 13(A), the welded portion 121 may have a pattern in which two triangular shapes in which non-welded portions 122 are formed are connected to each other.

As shown in Fig. 13(B), the welded portion 121 may have a pattern in a geometric shape that is continuously connected.

As shown in Fig. 13(C), the welded portion 121 may have a rhombic pattern.

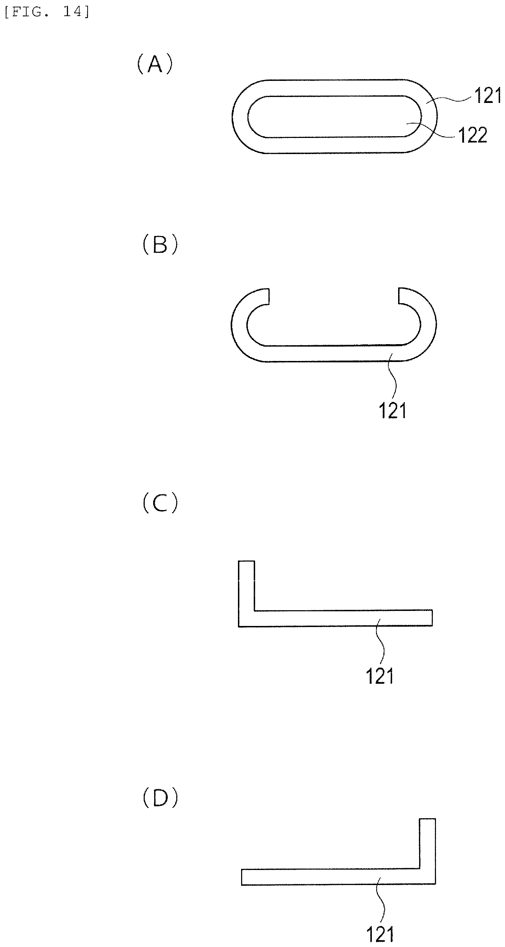

As shown in Fig. 14(A), the welded portion 121 may have an elliptical pattern.

As shown in Fig. 14(B), the welded portion 121 may have a pattern in which a part of an elliptical shape is missing.

As shown in Figs. 14(C) and 14(D), the welded portion 121 may have an L-shaped pattern.

In the description of the embodiment, the drug solution delivery apparatus is an application example of the medical device according to this disclosure, but the medical device is not limited to the drug solution delivery apparatus only. The medical device may be, for example, an inspection apparatus or the like including an inspection portion to be inserted into a living body. When the medical device is an inspection apparatus, a fixing region can be disposed around an insertion portion (hole or the like) formed in a housing through which the inspection portion is inserted into a living body.

The present application is based on Japanese Patent Application No. 2018-209904 filed on November 7, 2018, and the disclosure contents thereof are cited as a whole by reference.

### Reference Signs List

- 1: drug solution delivery apparatus (medical device),
- 10: housing,
- 11: bottom portion,
- 11a: placement surface,
- 11b: bottom surface (one surface),
- 12: fitting hole (hole portion),
- 13: side wall,
- 16: adhesion portion,
- 16a: upper surface
- 16b: bottom surface (one surface),
- 16c: adhesive portion,
- 17: insertion portion,
- 20: puncture needle,
- 30: delivery port portion,
- 35: puncture apparatus,
- 110, 110A, 110B, 110C: fixing region,
- 111: welded portion,
- 112: non-welded portion,
- 113: other welded portion,
- 114: other welded portion,
- 121: other welded portion,
- 122: non-welded portion,
- 131: other welded portion,
- 141: passage,
- 142, 143: groove portion,
- 145: vent hole.

## Claims

1. A medical device comprising:
a housing attachable to a living body;
a sheet-shaped adhesion portion provided on one surface on a living body side of the housing; and
a fixing region to which the housing and the adhesion portion are fixed, wherein
the housing includes a hole portion formed in the one surface of the housing and penetrating the one surface,
the adhesion portion includes an adhesive portion formed in at least a part of a surface facing the living body, and
the fixing region includes a welded portion formed near the hole portion and a non-welded portion formed at a position adjacent to the welded portion.

2. The medical device according to claim 1, wherein
the fixing region surrounds a periphery of the hole portion, and
the welded portion and the non-welded portion are alternately disposed along the outer periphery of the hole portion.

3. The medical device according to claim 1, wherein
the welded portion and the non-welded portion are formed in parallel linear patterns in which the welded portion and the non-welded portion are spaced apart from each other in the fixing region.

4. The medical device according to claim 1, wherein
the welded portion and the non-welded portion are formed in a lattice-shaped pattern in which the welded portion and the non-welded portion intersect with each other in the fixing region.

5. The medical device according to any one of claims 1 to 4, further comprising:
an auxiliary welded portion formed at a position different from the welded portion along one direction of the housing.

6. The medical device according to any one of claims 1 to 5, wherein
the housing includes an insertion portion for insertion of another member to be connected to the housing into the housing, and the medical device further comprises:
another welded portion in which the housing and the adhesion portion are welded to each other in a vicinity of the insertion portion.
